# EUROPÄISCHE PATENTSCHRIFT

(11) **EP 1 163 206 B1**
(45) Veröffentlichungstag und Bekanntmachung des Hinweises auf die Patenterteilung: **07.09.2005**
(21) Anmeldenummer: 00916969.9
(22) Anmeldetag: 17.03.2000
(51) Int. Cl.: C07C 209/62

(54) **VERFAHREN ZUR HYDROLYSE VON OPTISCH AKTIVEN AMIDEN**
METHOD FOR HYDROLYZING OPTICALLY ACTIVE AMIDES
PROCEDE PERMETTANT D'HYDROLYSER DES AMIDES OPTIQUEMENT ACTIFS

(30) Priorität: 24.03.1999 DE 19913256
(43) Veröffentlichungstag der Anmeldung: 19.12.2001
(73) Patentinhaber: BASF Aktiengesellschaft, 67056 Ludwigshafen (DE)
(72) Erfinder: DITRICH, Klaus, D-67161 Gönnheim (DE); LADNER, Wolfgang, D-67136 Fussgönheim (DE); MELDER, Johann-Peter, D-67459 Böhl-Iggelheim (DE)
(86) Internationale Anmeldenummer: PCT/EP2000/002380
(87) Internationale Veröffentlichungsnummer: WO 2000/056699

(56) Entgegenhaltungen:
- WO-A-95/08636
- WO-A-97/10201

## Beschreibung

Die vorliegende Erfindung betrifft ein neues Verfahren zur Spaltung von optisch aktiven Amiden.

Die hydrolytische Spaltung von optisch aktiven Amiden, die im Aminteil des Moleküls ein Chiralitätszentrum besitzen, ist nicht oder nur unter sehr aufwendigen Bedingungen so durchführbar, daß das Chiralitätszentrum erhalten bleibt.

Von Devant und Braun (Chem. Berichte 119, 2197-2207 (1986)) wird beschrieben, daß die Abspaltung von chiralen Aminen aus Acetamiden nicht ohne Zerstörung des Chiralitätszentrums möglich ist (Seite 2194). Weiterhin finden die Autoren, daß zahlreiche Versuche, die Amide alkalisch oder sauer zur Carbonsäure und optisch aktivem Amin zu hydrolysieren, erfolglos geblieben sind, und daß lediglich die Umsetzung mit Distickstofftetroxid nach White (J. Am. Chem. Soc. 77, 6008 (1955)) zum gewünschten Ergebnis führt. Diese Umsetzung mitN₂O₄ ist jedoch aufwendig und daher nicht für technische Verfahren geeignet.

In WO 95/08636 wird ein enzymatisches Verfahren zur Racematspaltung von optisch aktiven Aminen beschrieben, bei dem die Amine enantioselektiv mit einem Ester acyliert werden, dann die Mischung aus acyliertem Amin (Amid) und nicht-umgesetzten Amin getrennt wird und gewünschtenfalls aus dem acylierten Amin (Amid) das optisch aktive Amin durch Amidspaltung freigesetzt wird. Es werden jedoch keine Verfahrensparameter angegeben, bei denen die Amidspaltung durchgeführt werden kann.

In WO 97/10201 wird ein Verfahren zur Spaltung von optisch aktiven Amiden unter Erhalt des Chiralitätszentrums beschrieben. Dabei werden die Amide mit Alkali- oder Erdalkalihydroxid in Gegenwart eines Polyols oder Aminoalkohols hydrolysiert. Die verwendete Menge an Polyol oder Aminoalkohol beträgt 10 - 90, bevorzugt 30 - 80 Gew.-% bezogen auf die gesamte Mischung.

Es bestand die Aufgabe, das in WO 97/10201 beschriebene Verfahren bezüglich seiner Raum-Zeit-Ausbeuten noch weiter zu optimieren und die Kosten des Verfahrens abzusenken.

Diese Aufgabe wurde gelöst durch ein Verfahren zur Hydrolyse von optisch aktiven Amiden zu Carbonsäuren und optisch aktiven Aminen unter Erhalt des Chiralitätszentrums, wobei die Hydrolyse der Amide mit einem Alkali- oder Erdalkalihydroxid durchgeführt wird in Gegenwart von 5 - 30 Gew.-%, bezogen auf das eingesetzte Amid, eines Polyols oder eines Aminoalkohols.

Das erfindungsgemäße Verfahren eignet sich für praktisch alle Amide, die aus optisch aktiven primären oder sekundären Aminen herstellbar sind. Besonders geeignet ist es für Amide, deren Aminteil aus einem optisch aktiven Arylalkylamin besteht.

Besonders gut verläuft es bei primären Arylalkylaminen, beispielsweise solchen mit folgenden Strukturen: 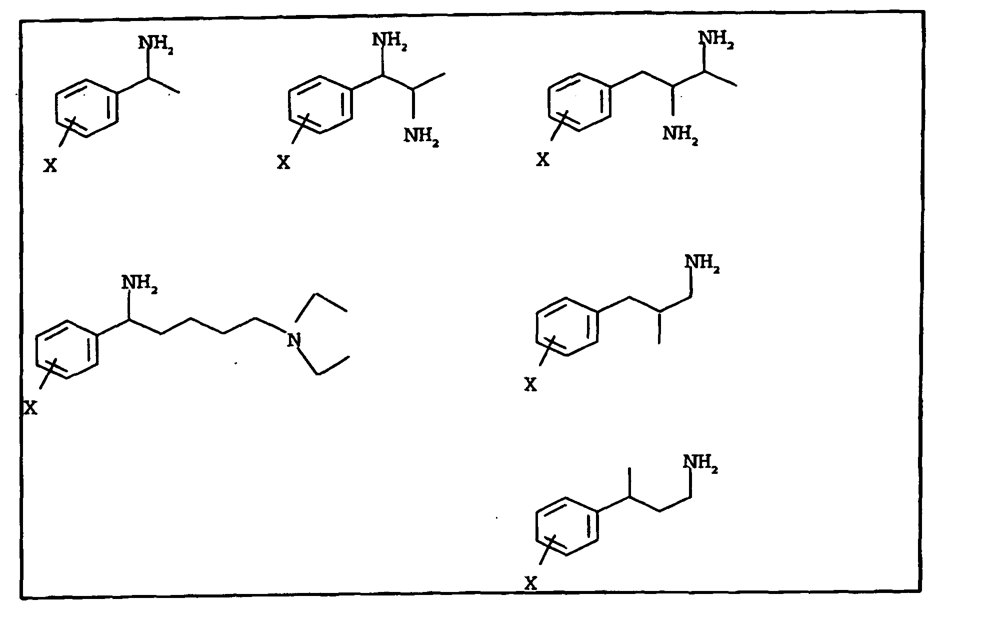 wobei X für alle gängigen Aromatensubstituenten, insbesondere Halogen, Nitro, Cyano, C₁-C₄-Alkyl, C₁-C₄-Alkoxy und C₁-C₄-Alkylthio, steht.

Des weiteren eignet sich das erfindungsgemäße Verfahren zur Spaltung von Amiden, deren Aminteil aus einem Aminoalkohole der allgemeinen Formel (I) besteht 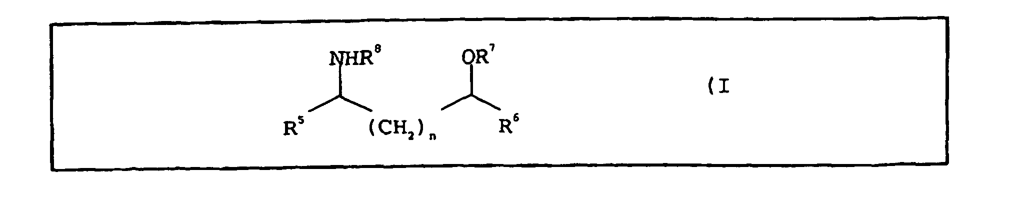 in der die substituenten folgende Bedeutung haben:
- R⁵, R⁶ =: unabhängig voneinander H, verzweigtes und unverzweigtes C₁-C₁₀-Alkyl, C₁-C₄-Alkoxycarbonyl, Phenyl, Phenyl-C₁-C₄-alkyl, wobei die Phenylgruppen durch Halogen, Nitro, Cyano, C₁-C₄-Alkyl, C₁-C₄-Alkoxy und C₁-C₄-Alkylthio substituiert sein können. Darüber hinaus können R⁵ und R⁶ über eine Kohlenstoffkette, die durch Sauerstoff, Schwefel oder Stickstoff unterbrochen und ihrerseits substituiert sein kann, zu einem Mono-, Bi- oder Tricyclus geschlossen sein
- R⁷ =: H, C₁-C₁₀-Alkyl, C₁-C₄-Alkoxycarbonyl
- R⁸ =: H, C₁-C₁₀-Alkyl
- n =: 0, 1 oder 2, bevorzugt 0 oder 1.

Sofern die durch OR⁷ bzw. NHR⁸ substituierten Kohlenstoffatome stereogene Zentren sind, bezieht sich das erfindungsgemäße Verfahren sowohl auf die syn- als auch die anti-Isomeren.

Als Beispiele für Aminoalkohole der obigen allgemeinen Struktur (I) seien genannt:
2-Amino-1-butanol; Ephedrin; Pseudoephedrin; Norephedrin; Norpseudoephedrin; tert. Leucinol; Phenylglycidol; 1,2-Diphenylaminoethanol; cis- und trans-2-Aminocyclopentanol; cis-und trans-1-Amino-2-hydroxyindan; cis- und trans-2-Aminocyclohexanol, Statin, 2-Hydroxy-3-amino-phenylpropionsäure.

Als bevorzugte Aminoalkohole sind zu nennen: cis- und trans-1-Amino-2-hydroxyindan.

Als Polyole können in dem erfindungsgemäßen Verfahren Glykole, z.B. Ethylenglykol und seine Monoether-, z.B. Monomethylglykol verwendet werden.

Weitere geeignete Polyole sind 1,2-Propandiol, 1,3-Propandiol, 1,2-Butandiol, 2,3-Butandiol, 2,4-Pentandiol, cis-und trans Cyclohexan-1,2-diol, cis-und trans Cyclohexan-1,4-diol, 2-Methyl-2,3-butandiol, 3-Methyl-2,4-pentandiol, 2,2-Dimethyl-1,3-propandiol, 1-Phenyl-1,2-ethandiol, 3-Methoxy-1,2-propandiol, 3-Phenox-1,2-propandiol, 3-Buten-1,2-diol, cis- und trans-2-Buten-1,4-diol, Triethanolamin, Triisopropanolamin.

Weiterhin können auch Polyalkylenglykole, bevorzugt Dialkylenglykole und deren Ether, insbesondere Diethylenglykol und Diglyme, als Polyole eingesetzt werden.

Bevorzugte Polyole sind Ethylenglykol und Diethylenglykol.

Geeignete Aminoalkohole für die erfindungsgemäße Amidspaltung sind Ethanolamin, Diethanolamin und Triethanolamin. Besonders bevorzugter Aminoalkohol ist Triethanolamin.

Die Polyole oder Aminoalkohole sollen in Wasser löslich oder mit Wasser homogen mischbar sein. Es können auch Mischungen verschiedener Polyole oder Aminoalkohole eingesetzt werden.

Die Polyole werden in einer Menge von 8 - 15 Gew.-%, bezogen auf das eingesetzte Amid, verwendet.

Ein weiterer notwendiger Bestandteil bei der erfindungsgemäßen Spaltung sind Alkali- oder Erdalkalihydroxide, insbesondere Natrium- und Kaliumhydroxid. Diese katalysieren die Hydrolyse ,werden jedoch auch durch die entstehende Säure neutralisiert, so daß man sie üblicherweise in einer Menge von 1-10 Äquivalenten bezogen auf Amid einsetzt.

Die Hydroxide können vorteilhaft in Form ihrer wäßrigen Lösungen eingesetzt werden, da bei der erfindungsgemäßen Spaltung ohnehin ein gewisser Wasseranteil benötigt wird. Der Wasseranteil beträgt in der Regel 5 - 90 Gew.-% bezogen auf das gesamte Lösungsmittel. Die erfindungsgemäße Spaltung wird bevorzugt bei Temperaturen 100 - 180°C durchgeführt.

Die erfindungsgemäße Hydrolyse der Amide kann sowohl diskontinuierlich (batch-Fahrweise) oder kontinuierlich durchgeführt werden.

Bei kontinuierlicher Fahrweise kann beispielsweise ein Schlaufenreaktor oder auch eine Rührkesselkaskade eingesetzt werden.

Der Reaktionsverlauf kann mit üblichen Methoden, beispielsweise durch Gaschromatographie, leicht verfolgt werden.

Nach erfolgter Hydrolyse kann das erhaltene Amin von der als Salz vorliegenden Carbonsäure abgetrennt und isoliert werden. Das geschieht bevorzugterweise durch Extraktion, wobei als Extraktionsmittel Ether wie Diethylether, Methyl-tert.-Butylether und Dibutylether, halogenierte Kohlenwasserstoffe wie Dichlormethan oder Trichlorethylen oder Kohlenwasserstoffe wie Pentan, Hexan, Cyclohexan, Benzol, Toluol und Xylol eingesetzt werden.

Eine ebenfalls bevorzugte Ausführungsform der Isolierung des Amins ist die Wasserdampfdestillation.

Eine besonders geeignete Ausführungsform der Erfindung besteht darin, daß man die Spaltung bei einer so hohen Temperatur ausführt, das das erhaltene Reaktionsprodukt (Amin) mit dem Wasserdampf überdestilliert und so gleich aus dem Reaktionsgemisch entfernt wird, während die Säure, die bei den alkalischen Bedingungen dissoziiert vorliegt, in der Vorlage zurückbleibt.

Das erfindungsgemäße Verfahren läßt sich mit gutem Erfolg als Teilschritt (Schritt 3) bei dem in WO 95/08636 beschriebenen Verfahren zur Racematspaltung von primären und sekundären Aminen einsetzen. Dieses Verfahren umfaßt die folgenden Schritte:
1. Umsetzung der racemischen Amine mit einem Ester, dessen Säurekomponente ein Fluor-, Stickstoff-, Sauerstoff-, Phosphor- oder Schwefelatom, gebunden an ein Kohlenstoffatom in alpha, beta oder gamma Position zum Carbonylkohlenstoffatom, trägt, unter spezifischer Katalyse mit einer Hydrolase,
2. Trennung des einen, enantioselektiv acylierten Amins vom nicht umgesetzten anderen Enantiomer des Amins,
3. anschließende Hydrolyse des acylierten Amins.

Die für dieses Verfahren geeigneten Ester sind solche, die in der Säurekomponente des Esters ein elektronenreiches Heteroatom, gebunden an ein Kohlenstoffatom, das sich in alpha, beta oder gamma Position zum Carbonylkohlenstoff befindet, tragen.

Das Heteroatom kann ein Fluor-, Stickstoff-, Sauerstoff-, Phosphor- oder Schwefelatom sein. Als Heteroatom ist Sauerstoff bevorzugt.

Das Heteroatom kann gegebenenfalls mit weiteren Gruppen, z.B. Alkylgruppen, verknüpft sein. Ist das Heteroatom beispielsweise Sauerstoff, so liegt eine Ethergruppe vor.

Die Alkoholkomponente des Esters kann aus verzweigten oder unverzweigten C₁- bis C₁₀-Alkoholen, die auch gegebenenfalls substituiert sein können, bestehen.

Besonders geeignete Alkoholkomponenten sind 2-Propanol, 2-Butanol, 2-Pentanol, 3-Pentanol, 3- Methyl-2-butanol, Cyclopentanol, Cyclohexanol, 2-Methylcyclohexanol, 1-Chlor-2-propanol, 1-Brom-2-Propanol, 4-Methyl-2-pentanol, 2,4-Dimethyl-3-pentanol, Cyclopropylethanol, 1-Phenylethanol, 1-Phenoxy-2-propanol, 1-Methoxy-2-propanol,cis-und trans 2-Methoxycyclohexanol, 1-Dimethylamino-2-propanol, 1-Buten-3-ol, 1-Butin-3-ol,1-Indanol, 2-Indanol, 3-Hydroxytetrahydrofuran, 5-Hydroxy-2-methyl-1,3-dioxan, 4-Hydroxypiperidin, (+) und (-)-Menthol, (+) und (-)-Isomenthol, Carfenol, Milchsäurenitril, Acetoncyanhydrin, Benzaldehydcyanhydrin, Pantolacton, Milchsäure-t-butylester, Acetonoxim-2-hydroxypropylether.

Weitere geeignete Alkoholkomponenten sind 1,2-Ethandiol, Glycerin, 1,2-Propandiol, 1,3-Propandiol, 1,2-Butandiol, 2,3-Butandiol, 2,4-Pentandiol, cis-und trans Cyclohexan-1,2-diol, cis-und trans Cyclohexan-1,4-diol, 2-Methyl-2,3 butandiol, 3-Methyl-2,4-pentandiol, 2,2-Dimethyl-1,3-propandiol,1-Phenyl-1,2-ethandiol, 3-Methoxy-1,2-propandiol, 3-Phenox-1,2-propandiol, 3-Chlor-1,2-propandiol, 3-Brom-1,2-Propandiol, 3-Buten-1,2-diol, cis- und trans-2-Buten-1,4-diol, Triethanolamin, Triisopropanolamin.

Besonders geeignete Ester sind solche mit der Struktur 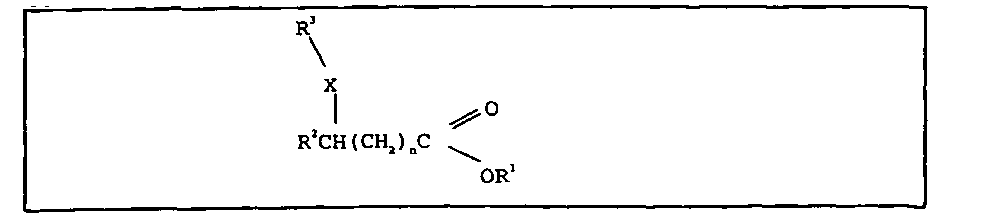 worin
- R¹ =: C₁-C₁₀-Alkyl,
- R² =: C₁-C₁₀-Alkyl, H
- R³ =: H, C₁-C₁₀-Alkyl, gegebenenfalls durch NH₂, OH, C₁-₄-Alkoxy oder Halogen substituiertes Phenyl,
- X =: O, S, NR⁴,
- R⁴ =: H, C₁-C₁₀-Alkyl, gegebenenfalls durch NH₂, OH, C₁-₄-Alkoxy oder Halogen substituiertes Phenyl,
- n =: 0,1 oder 2
bedeuten. Unter diesen sind die C₁-₄-Alkylester der C₁-₄-Alkoxyessigsäuren, beispielsweise der Methoxyessigsäure, bevorzugt. Ganz besonders bevorzugt sind die Methyl-, Ethyl-, n-Propyl-, i-Propyl-, n-Butyl-, i-Butyl-, tert. Butylester der Methoxyessigsäure.

Als Hydrolasen können in dem genannten Verfahren eine Vielzahl von Enzymen eingesetzt werden. Bevorzugt werden Proteasen und insbesondere Lipasen verwendet. Als Lipasen sind vor allem mikrobielle Lipasen gut geeignet, die beispielsweise aus Hefen oder Bakterien isolierbar sind. Besonders gut geeignet sind Lipasen aus Pseudomonas, z. B. Amano P oder die Lipase aus Pseudomonas spec. DSM 8246. Weitere besonders gut geeignete Hydrolasen sind die von Novo Nordisk (Enzyme Toolbox) kommerziell erhältlichen Enzyme, insbesondere die Lipasen SP 523, SP 524; SP 525, SP 526 und Novozym® 435.

Des weiteren können die Lipasen "Chirazyme L1 bis L8", welche kommerziell erhältlich sind (Boehringer Mannheim), vorteilhaft in dem erfindungsgemäßen Verfahren eingesetzt werden.

Das verwendete Enzym kann in nativer oder in immobilisierter Form eingesetzt werden.

Besonders gut eignet sich das immobilisierte Enzym Novozym® 435.

Als Lösungsmittel sind generell organische Lösungsmittel geeignet. Besonders gut verläuft die Reaktion in Ethern, beispielsweise in MTBE oder THF, oder in Kohlenwasserstoffen wie Hexan, Cyclohexan, Toluol oder halogenierten Kohlenwasserstoffen wie Methylenchlorid.

Die Umsetzung ist aber auch in Abwesenheit eines Lösungsmittels durchführbar.

Die Reaktion verläuft besonders gut, wenn die Lösungsmittel und Einsatzstoffe möglichst wasserfrei vorliegen.

Die Umsetzung des Esters mit dem racemischen Amin bzw. Aminoalkohol unter Enzymkatalyse wird üblicherweise bei Raumtemperatur ausgeführt. Die Reaktionszeiten dafür betragen je nach Substrat und Enzym-Menge 1 bis 48 Stunden. Sekundäre Amine/Aminoalkohole benötigen in der Regel längere Reaktionszeiten als primäre Amine/Aminoalkohole. Die geringere Reaktivität sekundärer Amine kann auch durch eine gegenüber primären Aminen erhöhte Menge an Katalysator ausgeglichen werden.

Pro Mol umzusetzendes Substrat werden 1 bis 6 Mol Ester bevorzugt zugesetzt, d.h. für 1 Mol racemisches Amin benötigt man 0,5 bis 3 Mol Ester.

Die zuzusetzende Menge an Enzym hängt von der Art der Hydrolase und der Aktivität der Enzympräparation ab. Die für die Reaktion optimale Enzymmenge kann leicht durch einfache Vorversuche ermittelt werden. In der Regel werden 1000 Units Lipase pro mMol Amin bzw. Aminoalkohol zugesetzt.

Der Reaktionsverlauf läßt sich leicht mit üblichen Methoden beispielsweise mittels Gaschromatographie verfolgen. Im Falle der Racematspaltung beendet man die Reaktion sinnvollerweise bei einem Umsatz von 50 % des racemischen Amins bzw. Aminoalkohols. Dies geschieht in der Regel durch Entfernen des Katalysators aus dem Reaktionsraum, beispielsweise durch Abfiltrieren des Enzyms. Wird die enantioselektive Amidierung in einem kontinuierlichen Modus durchgeführt, kann das Enzym in einem Durchflußreaktor zurückgehalten werden.

Durch die enantioselektive Umsetzung des racemischen Substrats mit dem Ester entsteht aus dem einen Enantiomer das entsprechend acylierte Produkt (Amid), während das andere Enantiomer unverändert bleibt. Das nun vorliegende Gemisch aus Amin und Amid läßt sich mit üblichen Methoden leicht trennen. Gut geeignet zur Trennung des Gemisches aus Amin und Amid sind beispielsweise Extraktions- oder Destillationsverfahren.

Die anschließende Spaltung des optisch aktiven Amids geschieht nach dem oben beschriebenen Verfahren.

Die folgenden Beispiele dienen der Veranschaulichung der Erfindung.

### Beispiel 1

Hydrolyse von N-1-(4-Chlorphenyl)-ethyl-methoxyacetamid

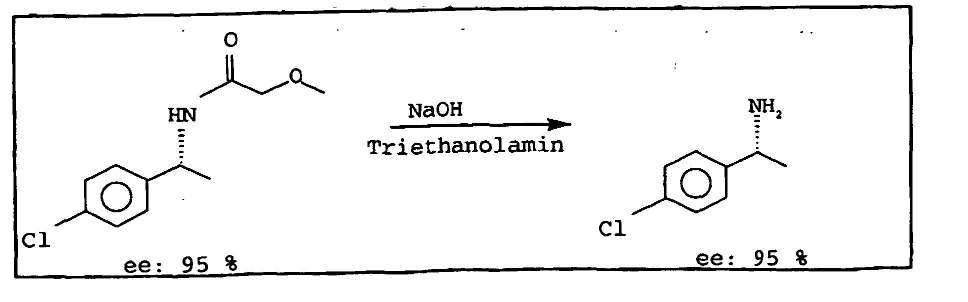

66 g Amid (0,29 mol) wurden bei 85°C mit 7,3 g Triethanolamin (11 Gew.-% bez. auf Amid) versetzt und auf 120°C erhitzt. Man gab 29,0 g (0,325 mol) 50%ige Natronlauge zu, rührte 2 Stunden bei 115-120°C nach und leitete dann Wasserdampf durch das Reaktionsgemisch. Als das Kopfdestillat klar überging, ließ man das gesammelte Kondensat auf Raumtemperatur abkühlen und trennte die untere organische Phase ab. Dieses Rohprodukt wurde im Vakuum destilliert. Man erhielt 41,5 g (92 %) R-1-(4-Chlorphenyl)ethylamin vom Kp: 141-144°C bei 110 mbar. Die optische Reinheit betrug 95 % ee (lt. GC).

### Beispiel 2

Hydrolyse von N-1-(4-Methylphenyl)-ethyl-methoxyacetamid

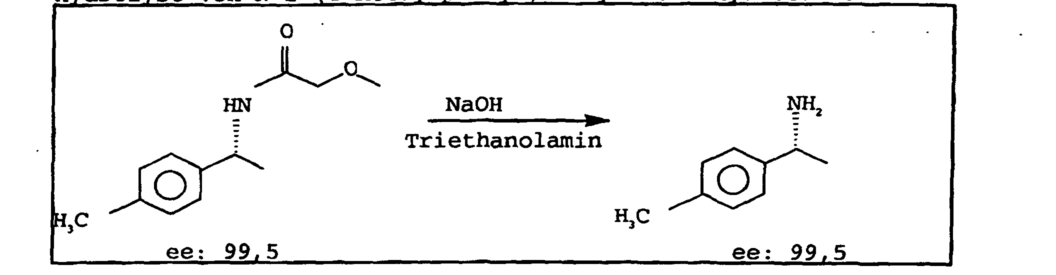

500 g (2,4 mol) R-N-1-(4-Methylphenyl)-ethyl-methoxyacetamid und 50 g Triethanolamin (10 Gew.-%, bezogen auf Amid) wurden auf 115°C erhitzt und mit 290 g (3,6 mol) 5o%iger Natronlauge versetzt. Die entstandene Suspension wurde 4 Stunden bei 115 - 120°C durchgerührt. Man gab 250 ml Wasser zu, ließ auf 95°C abkühlen, gab 500 ml Toluol zu und kühlte unter Rühren auf Raumtemperatur ab. Die Phasen wurden getrennt und die organische obere Phase im Vakuum destilliert. Zunächst ging ein Azeotrop aus Wasser und Toluol, dann Toluol und schließlich das Produkt R-1-(4-Methylphenyl)-ethylamin über. Man erhielt 287 g (88 %) Amin vom Kp: 98°C bei 24 mbar. Die optische Reinheit liegt bei 99,5 % ee (lt. GC).

## Patentansprüche

1. Verfahren zur Hydrolyse von optisch aktiven Amiden zu Carbonsäuren und optisch aktiven Aminen unter Erhalt des Chiralitätszentrums, wobei die Hydrolyse der Amide bei einer Temperatur zwischen 100 - 180 °C mit einem Alkali- oder Erdalkalihydroxid durchgeführt wird in Gegenwart von 8 - 15 Gew.%, bezogen auf das eingesetzte Amid, eines Polyols oder in Gegenwart von 5 - 30 Gew.-%, bezogen auf das eingesetzte Amid, eines Aminoalkohols.

2. Verfahren nach Anspruch 1, **dadurch gekennzeichnet, dass** als Polyol Ethylenglykol oder Diethylenglykol oder als Aminoalkohol Triethanolamin verwendet wird.

3. Verfahren nach Anspruch 1, **dadurch gekennzeichnet, dass** als Polyol Ethylenglykol oder Diethylenglykol verwendet wird.

4. Verfahren nach Anspruch 1, **dadurch gekennzeichnet, dass** die Hydrolyse kontinuierlich oder diskontinuierlich durchgeführt wird.

## Claims

1. A process for hydrolyzing optically active amides to carboxylic acids and optically active amines with retention of the center of chirality, where the hydrolysis of the amides is carried out at a temperature of between 100 - 180°C with an alkali metal or alkaline earth metal hydroxide in the presence of 8 - 15% by weight, based on the amide employed, of a polyol, or in the presence of 5 - 30% by weight, based on the amide employed, of an amino alcohol.

2. A process as claimed in claim 1, wherein ethylene glycol, diethylene glycol is used as polyol or triethanolamine is used as amino alcohol.

3. A process as claimed in claim 1, wherein ethylene glycol or diethylene glycol is used as polyol.

4. A process as claimed in claim 1, wherein the hydrolysis is carried out continuously or batchwise.

## Revendications

1. Procédé d'hydrolyse d'amides optiquement actifs en acides carboxyliques et amines optiquement actives en maintenant le centre de chiralité, dans lequel l'hydrolyse des amides est effectuée à une température comprise entre 100 et 180°C avec un hydroxyde alcalin ou alcalino-terreux, en présence de 8-15 % en poids, par rapport à l'amide mis en oeuvre, d'un polyol ou en présence de 5-30 % en poids, par rapport à l'amide mis en oeuvre, d'un aminoalcool.

2. Procédé suivant la revendication 1, **caractérisé en ce que**, comme polyol, on utilise de l'éthylèneglycol ou du diéthylèneglycol ou, comme aminoalcool, de la triéthanolamine.

3. Procédé suivant la revendication 1, **caractérisé en ce que**, comme polyol, on utilise de l'éthylèneglycol ou du diéthylèneglycol.

4. Procédé suivant la revendication 1, **caractérisé en ce que** l'hydrolyse est effectuée de manière continue ou discontinue.
